# FASCICULE DE BREVET EUROPEEN

(11) **EP 0 916 671 B1**
(45) Date de publication et mention de la délivrance du brevet: **30.01.2002**
(21) Numéro de dépôt: 98402806.8
(22) Date de dépôt: 13.11.1998
(51) Int. Cl.: C07D 471/04, A61K 31/44

(54) **Nouveaux inhibiteurs de métalloprotéases leur procédé de préparation et les compositions pharmaceutiques qui les contiennent**
Metalloproteaseinhibitoren, Verfahren zu ihrer Herstellung und diese enthaltende pharmazeutische Zubereitungen
Metalloprotease inhibitors, process for their preparation and pharmaceutical compositions containing them

(30) Priorité: 14.11.1997 FR 9714278
(43) Date de publication de la demande: 19.05.1999
(73) Titulaire: LES LABORATOIRES SERVIER, 92200 Neuilly sur Seine (FR)
(72) Inventeur: De Nanteuil, Guillaume, 92150 Suresnes (FR); Remond, Georges, 78000 Versailles (FR); Paladino, Joseph, 78700 Conflans Sainte Honorine (FR); Atassi, Ghanem, 92210 Saint Cloud (FR); Pierre, Alain, 78580 Les Alluets le Roi (FR); Tucker, Gordon, 75017 Paris (FR); Bonnet, Jacqueline, 75013 Paris (FR); Sabatini, Massimo, 92380 Garches (FR)

(56) Documents cités:
- EP-A- 0 803 505
- WO-A-97/18194

## Description

La présente invention concerne de nouveaux inhibiteurs de métalloprotéases, leur procédé de préparation et les compositions pharmaceutiques qui les contiennent.

A l'état physiologique, la synthèse des tissus connectifs est en équilibre dynamique avec la dégradation de la matrice extracellulaire. Cette dégradation est dûe à des protéases à zinc (métalloprotéases) secrétées par les cellules de la matrice existante : ce sont de façon non limitative les collagénases (MMP-1), les gélatinases ou collagénases de type IV (MMP-2, MMP-9) et les stromélysines (MMP-3).

A l'état normal, ces enzymes cataboliques sont régulées au niveau de leur synthèse et de leur sécrétion, ainsi qu'au niveau de leur activité enzymatique extracellulaire par des inhibiteurs naturels, comme l'α₂-macroglobuline ou les TIMP (Tissue Inhibitor of MetalloProteinase) qui forment des complexes inactifs avec les métalloprotéases.

Le point commun aux pathologies impliquant ces enzymes est un déséquilibre entre l'activité des enzymes activées et celle de leurs inhibiteurs naturels, avec pour conséquence une dégradation excessive des tissus.

La dégradation incontrôlée et accélérée des membranes par la résorption de la matrice extracellulaire catalysée par les métalloprotéases est un paramètre commun à plusieurs conditions pathologiques comme l'arthrite rhumatoïde, l'arthrose, l'invasion et la croissance tumorale, y compris la dissémination maligne et la formation de métastases, les ulcérations, l'athérosclérose, etc...

Récemment, le BB94, inhibiteur de métalloprotéases a montré une activité antitumorale en clinique où il s'est révélé actif sur les cancers de l'ovaire (Becket et al., DDT 1996, 1 (1), 16).

On peut donc attendre d'un inhibiteur de métalloprotéases qu'il restaure l'équilibre entre protéase et inhibiteur et de ce fait modifie de façon favorable l'évolution de ces pathologies.

Un certain nombre d'inhibiteurs de métalloprotéases ont été décrits dans la littérature. C'est le cas, plus particulièrement, des composés décrits dans les demandes de brevet WO 95/35275, WO 95/35276, EP 606046 ou WO 96/00214.

Les composés de l'invention, outre le fait qu'ils soient nouveaux, se sont révélés être des inhibiteurs de métalloprotéases ce qui les rend potentiellement utiles pour le traitement des cancers, des maladies rhumatismales comme l'arthrose et l'arthrite rhumatoïde, de l'athérosclérose, etc...

Plus spécifiquement, la présente invention concerne les composés de formule (I) : 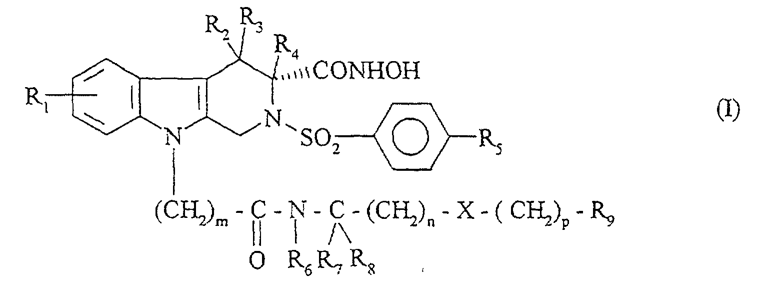 dans laquelle :
- m: représente un entier compris entre 1 et 4 inclus,
- n et p,: identiques ou différents, indépendamment l'un de l'autre, représentent un entier compris entre 0 et 4 inclus,
- x: représente un atome d'oxygène, de soufre ou une liaison simple,
- R₁: représente un atome d'hydrogène, un atome d'halogène, un groupement alkyle (C₁-C₆) linéaire ou ramifié, trihalogénoalkyle (C₁-C₆) linéaire ou ramifié, hydroxy, alkoxy (C₁-C₆) linéaire ou ramifié ou un groupement trihalogénoalkoxy (C₁-C₆) linéaire ou ramifié,
- R₂, R₃, R₄,: identiques ou différents, indépendamment l'un de l'autre, représentent un atome d'hydrogène ou un groupement alkyle (C₁-C₆) linéaire ou ramifié,
- R₅: représente un atome d'hydrogène, un atome d'halogène, un groupement alkoxy (C₁-C₆) linéaire ou ramifié, aryloxy ou un groupement hétéroaryloxy,
- R₆, R₇, R₈,: identiques ou différents, indépendamment l'un de l'autre, représentent un atome d'hydrogène, un groupement alkyle (C₁-C₆) linéaire ou ramifié, ou forment ensemble (R₆ et R₇ ou R₆ et R₈), avec l'atome d'azote portant le groupement R₆ et l'atome de carbone portant les groupements R₇ et R₈, un hétérocycle éventuellement substitué, le groupement restant (respectivement R₈ ou R₇) prenant alors une des définitions précédemment citées,
- R9: représente l'un quelconque des groupements suivants :
* -SO₃H,
* -CO₂R₁₀ dans lequel R₁₀ représente un atome d'hydrogène ou un groupement alkyle (C₁-C₆) linéaire ou ramifié,
* -CO-NR₁₁R₁₂ dans lequel R₁₁ et R₁₂, identiques ou différents, représentent un atome d'hydrogène, un groupement alkyle (C₁-C₆) linéaire ou ramifié, ou R₁₁ et R₁₂ forment ensemble, avec l'atome d'azote qui les porte, un hétérocycle éventuellement substitué,
* -NR₁₃R₁₄ dans lequel R₁₃ et R₁₄, identiques ou différents, représentent un atome d'hydrogène, un groupement alkyle (C₁-C₆) linéaire ou ramifié, ou R₁₃ et R₁₄ forment ensemble, avec l'atome d'azote qui les porte, un hétérocycle éventuellement substitué,
leurs isomères ainsi que leurs sels d'addition à un acide ou à une base pharmaceutiquement acceptable.

Etant entendu que :
- par groupement aryle, on comprend un groupement phényle, naphtyle, dihydronaphtyle, tétrahydronaphtyle, chacun de ces groupements étant éventuellement substitué par un ou plusieurs groupements identiques ou différents choisis parmi halogène, alkyle (C₁-C₆) linéaire ou ramifié, trihalogénoalkyle (C₁-C₆) linéaire ou ramifié, alkoxy (C₁-C₆) linéaire ou ramifié, trihalogénoalkoxy (C₁-C₆) linéaire ou ramifié ou hydroxy,
- par groupement hétéroaryle, on comprend un groupement aryle, éventuellement substitué par un ou plusieurs groupements, tels que définis précédemment, et contenant un, deux ou trois hétéroatomes, identiques ou différents, choisis parmi oxygène, azote ou soufre,
- par hétérocycle, on comprend un groupement mono ou bicyclique saturé ou insaturé, de 4 à 7 chaînons contenant un, deux ou trois hétéroatomes, identiques ou différents, choisis parmi oxygène, azote ou soufre, ledit hétérocycle pouvant être éventuellement substitué par un ou plusieurs groupements identiques ou différents choisis parmi halogène, alkyle (C₁-C₆) linéaire ou ramifié, alkoxy (C₁-C₆) linéaire ou ramifié, hydroxy, amino (éventuellement substitué par un ou deux groupements, identiques ou différents, alkyle (C₁-C₆) linéaire ou ramifié) ou par un hétérocycle,

Parmi les acides pharmaceutiquement acceptables, on peut citer à titre non limitatif les acides chlorhydrique, bromhydrique, sulfurique, phosphonique, acétique, trifluoroacétique, lactique, pyruvique, malonique, succinique, glutarique, fumarique, tartrique, maléique, citrique, ascorbique, oxalique, méthane sulfonique, camphorique, etc..

Parmi les bases pharmaceutiquement acceptables, on peut citer à titre non limitatif l'hydroxyde de sodium, l'hydroxyde de potassium, la triéthylamine, la tertbutylamine, etc...

L'invention s'étend également au procédé de préparation des composés de formule (I) caractérisé en ce que l'on utilise comme produit de départ un dérivé du D-tryptophane, sous forme racémique ou d'énantiomère pur, de formule (II) : 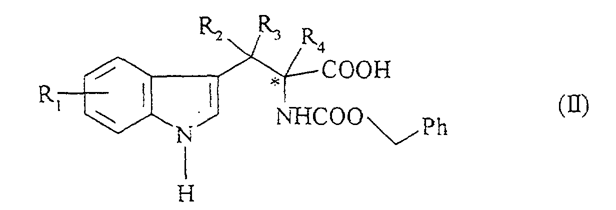 dans laquelle R₁, R₂, R₃ et R₄ sont tels que définis dans la formule (I), dont on substitue la fonction amine du cycle indole par un dérivé halogéné de formule (III) :

Z - (CH₂)ₘ - CO₂Ra (III)

dans laquelle m a la même définition que dans la formule (I), Z représente un atome d'halogène et Ra représente un groupement alkyle (C₁-C₆) linéaire ou ramifié, pour conduire aux composés de formule (IV) : 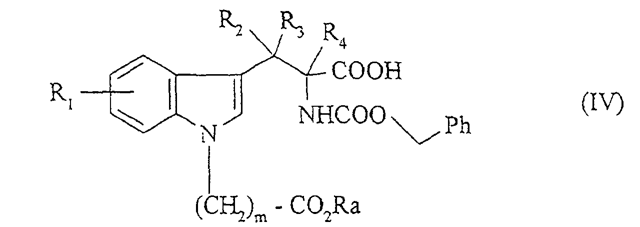 dans laquelle R₁, R₂, R₃, R₄, Ra et m sont tels que définis précédemment, composés de formule (IV) dont on déprotège la fonction amine de la chaîne latérale par une réaction d'hydrogénation catalytique pour conduire aux composés de formule (V) : 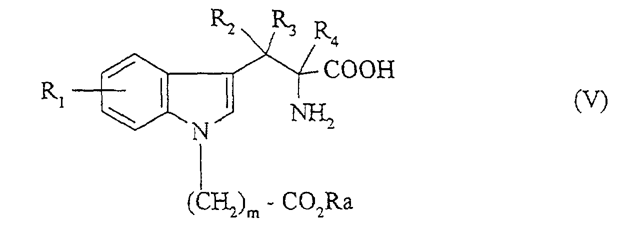 dans laquelle R₁, R₂, R₃, R₄, Ra et m sont tels que définis précédemment, que l'on cyclise, en présence d'un acide fort et de formaldéhyde, pour obtenir les composés de formule (VI) : 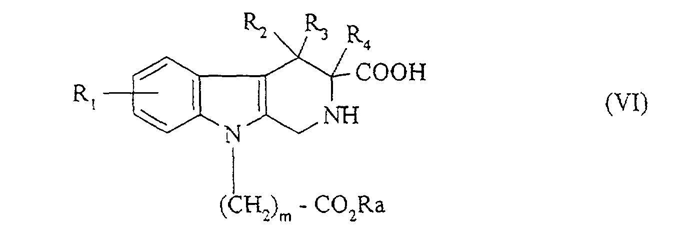 dans laquelle R₁, R₂, R₃, R₄, Ra et m sont tels que définis précédemment, composés de formule (VI) que l'on condense en milieu basique avec un dérivé halogéné de formule (VII) : 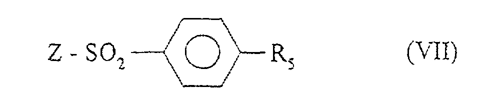 dans laquelle R₅ a la même signification que dans la formule (I) et Z représente un atome d'halogène,
pour conduire aux composés de formule (VIII) : 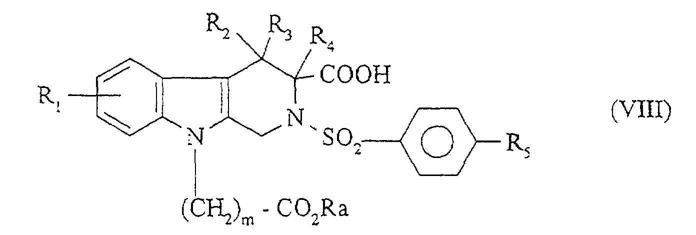 dans laquelle R₁, R₂, R₃, R₄, R₅, Ra et m sont tels que définis précédemment,
que l'on condense en milieu basique et en présence de dicyclohexylcarbodiimide avec une hydroxylamine-O-substituée de formule (IX) :

H₂N - O - R₁₅ (IX)

dans laquelle R₁₅ représente un groupement allyle ou benzyle,
pour obtenir les composés de formule (X) : 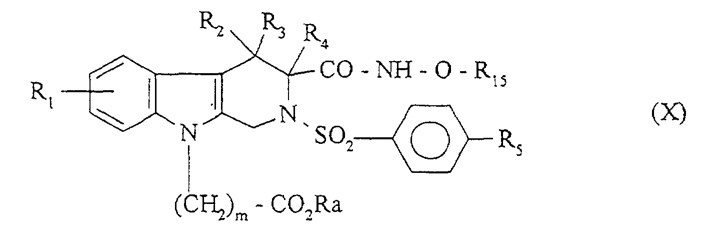 dans laquelle R₁, R₂, R₃, R₄, R₅, R₁₅, Ra et m sont tels que définis précédemment, dont on déprotège la fonction acide carboxylique, en présence par exemple d'acide trifluoroacétique, pour conduire aux composés de formule (XI): 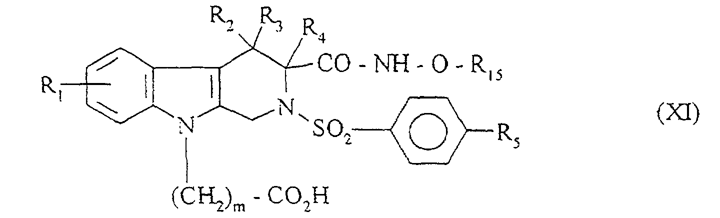 dans laquelle R₁, R₂, R₃, R₄, R₅, R₁₅ et m sont tels que définis précédemment,
que l'on condense avec une amine de formule (XII) : 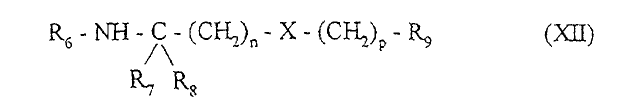 dans laquelle R₆, R₇, R₈, R₉, n et p ont la même définition que dans la formule (I), pour conduire aux composés de formule (XIII) : 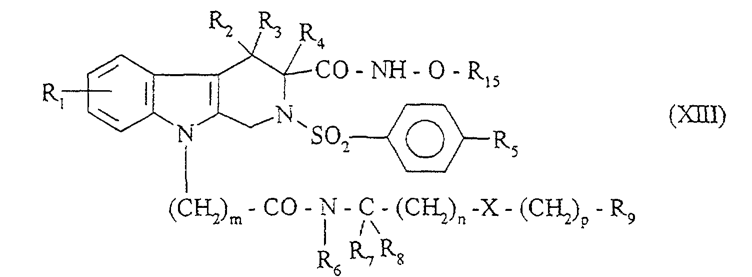 dans laquelle R₁, R₂, R₃, R₄, R₅, R₆, R₇, R₈, R₉ et R₁₅ sont tels que définis précédemment, ainsi que m, n et p, composés de formule (XIII) dont la fonction hydroxylamine est déprotégée, pour conduire aux composés de formule (I) : 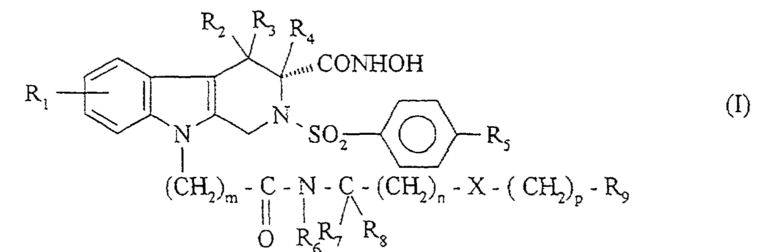 dans laquelle R₁, R₂, R₃, R₄, R₅, R₆, R₇, R₈, R₉, m, n et p sont tels que définis précédemment, composés de formule (I) que l'on purifie, le cas échéant, selon une technique classique de purification, dont on sépare éventuellement les isomères selon une technique classique de séparation et que l'on transforme, si on le souhaite, en ses sels d'addition à un acide ou à une base pharmaceutiquement acceptable.

Les composés de formule (II), (III), (VII), (IX) et (XII) sont soit des produits commerciaux, soit obtenus selon les méthodes classiques de la synthèse organique.

L'invention s'étend aussi aux compositions pharmaceutiques renfermant comme principe actif au moins un composé de formule (I), ses isomères optiques ou un de ses sels d'addition à un acide ou à une base pharmaceutiquement acceptable, seul ou en combinaison avec un ou plusieurs excipients inertes, non toxiques pharmaceutiquement acceptables. Parmi les compositions pharmaceutiques selon l'invention, on pourra citer plus particulièrement celles qui conviennent pour l'administration orale, parentérale (intraveineuse, intramusculaire ou sous-cutanée), nasale, rectale, per ou transcutannée, perlinguale, oculaire ou respiratoire, et notamment les comprimés simples ou dragéifiés, les comprimés sublinguaux, les gélules, les capsules, les suppositoires, les crèmes, les pommades, les gels dermiques, les préparations injectables, les suspensions buvables, les aérosols, les gouttes oculaires ou nasales, etc...

La posologie utile est adaptable selon la nature et la sévérité de l'affection, la voie d'administration, la prise de traitements éventuels associés, ainsi que l'âge et le poids du patient. Cette posologie varie de 0,01 g à 2 g en une ou plusieurs prises par jour.

Les exemples suivants illustrent l'invention mais ne la limitent en aucune façon.

Les produits de départ utilisés sont des produits connus ou préparés selon des modes opératoires connus

Les préparations A à G conduisent à des intermédiaires de synthèse, utiles pour la préparation des composés de l'invention.

Les structures des composés décrits dans les exemples et les préparations ont été déterminées selon les techniques spectrophotométriques usuelles (infrarouge, RMN, spectrométrie de masse, ...).

### EXEMPLE 1 : Chlorhydrate de 2-[(4-méthoxyphényl)sulfonyl]-9-[(3-morpholin-4-ylpropylcarbamoyl)méthyl]-2,3,4,9-tétrahydro-1H-β-carboline-(3R)-(N-hydroxy)carboxamide

### Stade A : 1-[(tert-Butoxycarbonyl)méthyl]-Nα-benzyloxycarbonyl-D-Tryptophane

A une solution de 100 g de Nα-benzyloxycarbonyl-D-Tryptophane dans un litre de tétrahydrofurane anhydre sont additionnés, goutte à goutte, à -78°C sous atmosphère inerte, 600 ml d'une solution de NaHMDS (sel de sodium de 1,1,1,3,3,3-Hexaméthyldisilazane) (1M) dans le tétrahydrofurane. Après une heure, la température est ramenée à 0°C et une solution de 64 g de bromoacétate de tert-butyle dans 50 ml de tétrahydrofurane anhydre est additionnée goutte à goutte. La réaction est alors ramenée à température ambiante, puis le solvant est évaporé sous vide. Le résidu est dilué dans du dichlorométhane et la solution est acidifiée par addition d'une solution aqueuse 4N d'acide chlorhydrique. Après extraction au dichlorométhane, les phases organiques sont séchées sur sulfate de sodium puis concentrées sous vide. On obtient 136 g du produit attendu.

### Stade B : 1-[(tert-Butoxycarbonyl)méthyl]-D-Tryptophane

Les 136 g de produit obtenus dans le stade A sont dilués dans 1,5 litres de méthanol et sont hydrogénés en présence de 5 g de Pd/C 10 % sous une pression en H₂ de 1,2 bars. Après 12 heures de réaction à température ambiante la solution est filtrée, puis concentrée sous vide. On obtient 87 g du produit attendu sous forme d'une poudre beige.
*Point de fusion : 260°C*

### Stade C : Acide 9-[(tert-butoxycarbonyl)méthyl]-2,3,4,9-tétrahydro-β-carboline-(3R)-carboxylique

A une suspension de 85 g du produit du stade B dans 600 ml d'eau sont additionnés 130 ml d'une solution 0,1 N d'acide sulfurique puis goutte à goutte 130 ml d'une solution de formaldéhyde à 37 %. Après 48 heures de réaction à température ambiante, le mélange réactionnel est filtré, lavé à l'eau puis séché sur P₂O₅. On obtient 82,6 g d'un solide blanc correspondant au produit attendu.
*Point de fusion : 230°C*

### Stade D : Acide 9-[(tert-butoxycarbonyl)méthyl]-2-[(4-méthoxyphényl)sulfonyl]-2,3,4,9-tétrahydro-1H-β-carboline-(3R)-carboxylique

A une solution de 80 g de produit du stade C dans 1,5 litres de dioxane sont additionnés 750 ml d'eau puis 85 ml de triéthylamine. Le mélange réactionnel est refroidi à 0°C et 60 g de chlorure de 4-Méthoxybenzènesulfonique dilués dans 100 ml de dioxane sont additionnés goutte à goutte La réaction est alors ramenée à température ambiante et l'agitation est maintenue pendant 12 heures. Après évaporation sous vide du dioxane, la solution résiduelle est diluée dans l'eau puis acidifiée par addition d'une solution d'acide chlorhydrique 4N, et extraite au dichlorométhane. Les phases organiques rassemblées sont ensuite lavées à l'eau, puis par une solution saturée au NaCl, séchées sur sulfate de sodium et concentrées sous vide. Le résidu est cristallisé dans du pentane, permettant d'obtenir 115,2 g du produit souhaité sous forme d'une poudre blanche.
*Point de fusion : 150°C*

### Stade E : 9-[(tert-Butoxycarbonyl)méthyl]-2-[(4-méthoxyphényl)sulfonyl]-2,3,4,9-tétrahydro-1H-β-carboline-(3R)-(N-allyloxy)carboxamide

A une solution de 100 g de composé du stade D dans 1,5 litres de diméthylformamide sont additionnés à 0°C, 24,1 g de O-allylhydroxylamine, 27 g d'hydroxybenzotriazole, 41,2 g de dicyclohexylcarbodiimide et 31 ml de triéthylamine. Après 12 heures d'agitation à température ambiante, le mélange réactionnel est concentré sous vide. Le résidu est dilué dans l'acétate d'éthyle, puis lavé successivement par une solution aqueuse à 10 % d'hydrogénocarbonate de sodium, par de l'eau et par une solution saturée au chlorure de sodium. La phase organique est ensuite séchée sur sulfate de sodium et évaporée. On obtient ainsi 120 g du produit attendu sous forme d'huile.

### Stade F : Acide 2-[(4-méthoxyphényl)sulfonyl]-2,3,4,9-tétrahydro-1H-β-carboline-(3R)-[(N-allyloxy)-carboxamide]-9-éthanoïque

A une solution de 50 g du composé du stade E, dans 500 ml de dichlorométhane anhydre, sont additionnés à 0°C 70 ml d'acide trifluoroacétique. Après 12 heures à température ambiante, le mélange réactionnel est concentré sous vide et le résidu obtenu est cristallisé dans de l'éther éthylique. On obtient ainsi 30,8 g de produit désiré sous forme d'un solide beige.
*Point de fusion : 160°C*

### Stade G : 2-[(4-Méthoxyphényl)sulfonyl]-9-[(3-morpholin-4-yl-propyl carbamoyl)-méthyl]-2,3,4,9-tétrahydro-1H-β-carboline-(3R)-(N-allyloxy)carboxamide

A une solution de 3,5 g du composé du stade F dans 50 ml de diméthylformamide sont additionnés à 0°C, 1 ml de N-(3-aminopropyl)morpholine, 0,95 g d'hydroxybenzotriazole et 1,5 g de dicyclohexylcarbodiimide. Après 12 heures à température ambiante, le mélange réactionnel est d'abord filtré afin d'éliminer l'excès de dicyclohexylcarbodiimide, puis concentré sous vide. Le résidu est dilué dans du dichlorométhane, lavé trois fois par de l'eau puis par une solution saturée au chlorure de sodium ; la phase organique est alors séchée sur sulfate de sodium, puis évaporée sous pression réduite. Le produit attendu est alors isolé par chromatographie sur gel de silice (éluant : dichlorométhane/méthanol/ hydroxyde d'ammonium : 95/5/0,5).

### Stade H : Chlorhydrate de 2-[(4-méthoxyphényl)sulfonyl]-9-[(3-morpholin-4-ylpropyl-carbamoyl)méthyl]-2,3,4,9-tétrahydro-1H-β-carboline-(3R)-(N-hydroxy)carboxamide

A une solution de 2,5 g du composé obtenu dans le stade G dans 50 ml de dichlorométhane sont additionnés à température ambiante et sous atmosphère inerte 0,7 ml d'acide acétique et 0,14 g de Pd (PPh₃)₂Cl₂. Après 5 minutes, 2,3 ml d'hydrure de tri-n-butylétain sont ajoutés et la réaction est maintenue à température ambiante pendant une heure, puis est évaporée à sec. Le résidu est alors dilué dans de l'hexane. Il se forme un précipité qui est filtré, dilué dans une solution aqueuse contenant de l'acide chlorhydrique ; celle-ci est alors lavée à l'éther puis à l'hexaae. Après filtration et lyophilisation, 2 g du produit attendu sont obtenus.

| *Microanalyse élémentaire :* | | | | | |
|---|---|---|---|---|---|
| | *C* | *H* | *N* | *Cl* | *S* |
| *% calculé* | *54,06* | *5,83* | *11,26* | *5,70* | *5,15* |
| *% trouvé* | *54,28* | *5,98* | *11,29* | *5,88* | *4,87* |

### EXEMPLE 2 : Acétate de 2-[(4-méthoxyphényl)sulfonyl]-9-[(2-morpholon-4-yl-éthyl carbamoyl)méthyl]-2,3,4,9-tétrahydro-1H-β-carboline-(3R)-(N-hydroxy)carboxamide

Le produit attendu est obtenu selon le même procédé décrit dans l'exemple 1 en utilisant au stade G la N-(2-aminopropyl)morpholine comme réactif.

| *Microanalyse élémentaire :* | | | | |
|---|---|---|---|---|
| | *C* | *H* | *N* | *S* |
| *% calculé* | *55,14* | *5,90* | *11,09* | *5,08* |
| *% trouvé* | *54,79* | *6,06* | *10,89* | *4,99* |

### EXEMPLE 3 : Chlorhydrate de 2-[(4-méthoxyphényl)sulfonyl]-9-[(4-morpholin-4-ylbutyl carbamoyl)méhtyl]-2,3,4,9-tétrahydro-1H-β-carboline-(3R)-(N-hydroxy)carboxamide

Le produit attendu est obtenu selon le même procédé décrit dans l'exemple 1 en utilisant au stade G la N-(4-aminobutyl)morpholine comme réactif.

| *Microanaxlyse élémentaire :* | | | | | |
|---|---|---|---|---|---|
| | *C* | *H* | *N* | *Cl* | *S* |
| *% calculé* | *54,75* | *6,02* | *18,01* | *5,57* | *5,04* |
| *% trouvé* | *54,53* | *6,27* | *10,58* | *5,75* | *5,23* |

### EXEMPLE 4 : Chlorhydrate de 2-[(4-méthoxyphényl)sulfonyl]-3-méthyl-9-[(2-morpholin-4-yl-éthyl carbamoyl)-méthyl]-2,3,4,9-tétrahydro-1H-β-carboline-(3R)-(N-hydroxy)carboxamide

Le produit attendu est obtenu selon le même procédé décrit dans l'exemple 1 en utilisant au stade A, en tant que substrat, l'acide 2-[(benzyloxycarbonyl)amino]-2-méthyl-3-(1H-3-indolyl)-(2R)-propanoique, et au stade G le réactif utilisé dans l'exemple 2.

### EXEMPLE 5 : Chlorhydrate de 2-[(4-méthoxyphényl)sulfonyl]-3-méthyl-9-[(3-morpholin-4-yl-propyl-carbamoyl)-méthyl]-2,3,4,9-tétrahydro-1H-β-carboline-(3R)-(N-hydroxy)carboxamide

Le produit attendu est obtenu selon le même procédé décrit dans l'exemple 1 en utilisant au stade A, en tant que substrat, le composé utilisé à ce stade dans l'exemple 4.

### EXEMPLE 6 : Chlorhydrate de 2-[(4-méthoxyphényl)sulfonyl]-4,4-diméthyl-9-[(2-morpholin-4-yl-éthyl carbamoyl)-méthyl]-2,3,4,9-tétrahydro-1H-β-carboline-(3R)-(N-hydroxy) carboxamide

Le produit attendu est obtenu selon le même procédé décrit dans l'exemple 1 en utilisant au stade A, en tant que substrat, l'acide 2-[(benzyloxycarbonyl)amino]-3-méthyl-3-(lH-3-indolyl)-(2R)-butanoïque, et au stade G le réactif utilisé dans l'exemple 2.

### EXEMPLE 7 : Chlorhydrate de 2-[(4-méthoxyphényl)sulfonyl]-4,4-diméthyl-9-[(3-morpholin-4-yl-propyl-carbamoyl)-méthyl]-2,3,4,9-tétrahydro-1H-β-carboline-(3R)-(N-hydroxy)carboxamide

Le produit attendu est obtenu selon le même procédé décrit dans l'exemple 1 en utilisant au stade A le substrat utilisé dans l'exemple 6.

### EXEMPLE 8 : 2-{[4-(4Pyridyloxy)phényl]sulfonyl}-9-[(2-morpholin-4-yl-éthylcarbamoyl)-méthyl]-2,3,4,9-tétrahydro-1H-β-carboline-(3R)-(N-hydroxy)carboxamide

Le produit attendu est obtenu selon le même procédé décrit dans l'exemple 1 en utilisant au stade D le chlorure de 4-(4-pyridyloxy)-benzènesulfonique comme réactif, et au stade G le réactif de l'exemple 2.

### EXEMPLE 9 : Chlorhydrate de 2-{[4(4-Pyridyloxy)phényl]sulfonyl}-9-[(3-morpholin-4-yl-propylcarbamoyl)-méthyl]-2,3,4,9-tétrahydro-1H-β-carboline-(3R)-(N-hydroxy)carboxamide

Le produit attendu est obtenu selon le même procédé décrit dans l'exemple 1 en utilisant au stade D le réactif de l'exemple 8.

### EXEMPLE 10 : Chlorhydrate de 2-[(4-méthoxyphényl)sulfonyl]-9-(2-[1,4']-bipipéridinyl-1'-yl-2-oxo-éthyl)-2,3,4,9-tétrahydro-1H-β-carboline-(3R)-(N-hydroxy)carboxamide

Le produit attendu est obtenu selon le même procédé décrit dans l'exemple 1 en utilisant au stade G la [1,4']-bipipéridine comme réactif.

| *Microanalyse élémentaire :* | | | | | |
|---|---|---|---|---|---|
| | *C* | *H* | *N* | *Cl* | *S* |
| *% calculé* | *57,62* | *6,24* | *10,84* | *5,49* | *4,96* |
| *% trouvé* | *57,58* | *6,13* | *10,49* | *5,72* | *4,86* |

### EXEMPLE 11 : Chlorhydrate de 2-[(4-méthoxyphényl)sulfonyl]-9-{[2-(2-morpholin-4-yl-éthylsufanyl)-éthylcarbamoyl]-méthyl}-2,3,4,9-tétrahydro-1H-β-carboline-(3R)-(N-hydroxy)carboxamide

Le produit attendu est obtenu selon le même procédé décrit dans l'exemple 1 en utilisant comme réactif au stade G la 2-[(2-morpholinoéthyl)sulfanyl]éthylamine.

| *Microanalyse élémentaire :* | | | | | |
|---|---|---|---|---|---|
| | *C* | *H* | *N* | *Cl* | *S* |
| % *calculé* | *52.13* | *5,73* | *10,48* | *5,31* | *9,60* |
| % *trouvé* | *52,80* | *5,80* | *10,33* | *5,20* | *9,34* |

### EXEMPLE 12 : Chlorhydrate de 2-[(4-méthoxyphényl)sulfonyl]-9-{[3-(3-morpholin-4-yl-propylsulfanyl)-propyl carbamoyl]-méthyl}-2,3,4,9-tétrahydro-1H-β-carboline-(3R)-(N-hydroxy)carboxamide

Le produit attendu est obtenu selon le même procédé décrit dans l'exemple 1 en utilisant au stade G la 3-[(3-morpholinopropyl)sulfanyl]propylamine, comme réactif.

| *Microanalyse élémentaire :* | | | | | |
|---|---|---|---|---|---|
| | *C* | *H* | N | *Cl* | *S* |
| *% calculé* | *53,48* | *6*,*08* | *10*,*06* | *5*,*09* | *9*,*21* |
| *% trouvé* | *53,10* | *6,04* | *9,60* | *5,05* | *8,95* |

### EXEMPLE 13 : Chlorhydrate de 2-[(4-méthoxyphényl)sulfonyl]-9-{[2-(3-morpholin-4-yl-propylsulfanyl)-éthylcarbamoyl]méthyl}-2,3,4,9-tétrahydro-1H-β-carboline-(3R)-(N-hydroxy)carboxamide

Le produit attendu est obtenu selon le même procédé décrit dans l'exemple 1 en utilisant au stade G la 2-[(3-morpholinopropyl)sulfanyl]éthylamine comme réactif.

| *Microanalyse élémentaire :* | | | | | |
|---|---|---|---|---|---|
| | *C* | *H* | *N* | *Cl* | *S* |
| *% calculé* | *52,81* | *5,91* | *10,26* | *5,20* | *9,40* |
| *% trouvé* | *52,88* | *5,79* | *9,98* | *5,25* | *9,06* |

### EXEMPLE 14 : Chlorhydrate de 2-[(4-méthoxyphényl)sulfonyl]-9-{[3-(2-morpholin-4-yl-éthylsulfanyl)-propyl carbamoyl]-méthyl}-2,3,4,9-tétrahydro-1H-β-carboline-(3R)-(N-hydroxy)carboxamide

Le produit attendu est obtenu selon le même procédé décrit dans l'exemple 1 en utilisant au stade G la 3-[(2-morpholinoéthyl)sulfanyl]propylamine comme réactif.

| *Microanalyse élémentaire :* | | | | | |
|---|---|---|---|---|---|
| | *C* | *H* | *N* | *Cl* | *S* |
| *% calculé* | *52,81* | *5,91* | *10,26* | *5,20* | *9,40* |
| *% trouvé* | *53,35* | *5,88* | *10,12* | *5,21* | *9,11* |

### EXEMPLE 15 : Chlorhydrate de 2-[(4-méthoxyphényl)sulfonyl]-9-[N-méthyl-(2-morpholin-4-yl-éthylcarbamoyl)-méthyl]-2,3,4,9-tétrahydro-1H-β-carboline-(3R)-(N-hydroxy)carboxamide

Le produit attendu est obtenu selon le même procédé décrit dans l'exemple 1 en utilisant a stade G la 4-(2-(méthylamino)éthyl)morpholine comme réactif.

| *Microanalyse élémentaire :* | | | | | |
|---|---|---|---|---|---|
| | *C* | *H* | *N* | *Cl* | *S* |
| *% calculé* | *54,06* | *5,83* | *11,26* | *5,70* | *5,15* |
| *% trouvé* | *54,32* | *5,81* | *10,86* | *5,81* | *4,68* |

### EXEMPLE 16 : 2-[(4-Méthoxyphényl)sulfonyl]-9-[(2-sulfonic éthyl carbamoyl)-méthyl]-2,3,4,9-tétrahydro-1H-β-carboline-(3R)-(N-hydroxy) carboxamide

Le produit attendu est obtenu selon le même procédé décrit dans l'exemple 1 en utilisant au stade G l'acide 2-aminvéthyl sulfonique comme réactif.
***Spectrométrie de masse : [M-H]***^{***-***} ***= 565***

### EXEMPLE 17 : Chlorhydrate de 2-[(4-méthoxyphényl)sulfonyl]-9-[(1,1-diméthyl-(2-morpholin-4-yl)-éthyl carbamoyl)-méthyl]-2,3,4,9-tétrahydro-1H-β-carboline-(3R)-(N-hydroxy)carboxamide

Le produit attendu est obtenu selon le même procédé décrit dans l'exemple 1 en utilisant au stade G la N-[(2-amino-2-méthyl)propyl]morpholine comme réactif.

| *Microanalyse élémentaire :* | | | | | |
|---|---|---|---|---|---|
| | *C* | *H* | *N* | *Cl* | *S* |
| *% calculé* | *54,75* | *6,02* | *11,01* | *5,57* | *5,04* |
| *% trouvé* | *55,16* | *6,22* | *11,17* | *5,36* | *4,78* |

### EXEMPLE 18 : Dichlorhydrate de 6-hydroxy-2-[(4-méthoxyphényl)sulfonyl]-9-[(3-carboxypropyl-carbamoyl)-méthyl]-2,3,4,9-tétrahydro-1H-β-carboline-(3R)-(N-hydroxy)carboxamide

Le produit attendu est obtenu selon le même procédé décrit dans l'exemple 1 en utilisant dans le stade A le N-benzyloxycarbonyl-5-hydroxy-D-tryptophane comme substrat et dans le stade G l'acide 4-amino-butanoïque comme réactif.

### EXEMPLE 19 : Chlorhydrate de 6-méthoxy-2-[(4-méthoxyphényl)sulfonyl]-9-[(3-carbamoylpropyl-carbamoyl)-méthyl]-2,3,4,9-tétrahydro-1H-β-carboline-(3R)-(N-hydroxy)carboxamide

Le produit attendu est obtenu selon le même procédé décrit dans l'exemple 1 en utilisant dans le stade A le N-benzyloxycarbonyl-5-méthoxy-D-tryptophane comme substrat et dans le stade G la 4-amino-butylamide.

### EXEMPLE 20 : Chlorhydrate de 6-méthoxy-2-[(4-méthoxyphényl)sulfonyl]-9-{[(4-(N,N-diméthyl)carbamoyl)-butyl-carbamoyl]-méthyl}-2,3,4,9-tétrahydro-1H-β-carboline-(3R)-(N-hydroxy)carboxamide

Le produit attendu est obtenu selon le même procédé décrit dans l'exemple 1 en utilisant au stade A le substrat pris dans l'exemple 19 pour cette étape et en utilisant au stade G la N,N-diméthyl-5-amino-pentylamide comme réactif.

### EXEMPLE 21 : Chlorhydrate de 6-méthoxy-2-[(4-méthoxyphényl)sulfonyl]-9-{[3-(N-éthyl)carbamoyl)-propyl-carbamoyl]-méthyl}-2,3,4,9-tétrahydro-1H-β-carboline-(3R)-(N-hydroxy)carboxamide

Le produit attendu est obtenu selon le même procédé décrit dans l'exemple 1 en utilisant au stade A le substrat pris dans l'exemple 19 pour cette étape, et en utilisant au stade G la N-éthyl-4-amino-butylamide comme réactif

### EXEMPLE 22 : Ddichlorhydrate de 6-hydroxy-2-[(4-méthoxyphényl)sulfonyl]-9-[(4-amino-butyl-carbamoyl)-méthyl]-2,3,4,9-tétrahydro-1H-β-carboline-(3R)-(N-hydroxy)carboxamide

Le produit attendu est obtenu selon le même procédé décrit dans l'exemple 1 en utilisant au stade A le substrat pris dans l'exemple 18 pour cette étape et en utilisant au stade G la 1,4-butyldiamine comme réactif.

### EXEMPLE 23 : 6-Méthoxy-2-[(4-méthoxyphényl)sulfonyl]-9-{[6-(N,N-diméthyl)aminohexyl-carbamoyl]-méthyl}-2,3,4,9-tétrahydro-1H-β-carboline-(3R)-(N-hydroxy)carboxamide

Le produit attendu est obtenu selon le même procédé que dans l'exemple 1 en utilisant au stade A le substrat pris dans l'exemple 19 pour cette étape et en utilisant au stade G la N1,N1-diméthyl-1,6-hexyldiamine comme réactif.

### EXEMPLE 24 : Chlorhydrate de 6-méthoxy-2-[(4-méthoxyphényl)sulfonyl]-9-{[2-(N-propyl)amino-éthyl-carbamoyl]-méthyl}-2,3,4,9-tétrahydro-1H-β-carboline-(3R)-(N-hydroxy)carboxamide,

Le produit attendu est obtenu selon le même procédé décrit dans l'exemple 1 en utilisant au stade A le substrat pris dans l'exemple 19 pour cette étape, et en utilisant au stade G la N1-propyl-1,2-éthyldiamine comme réactif.

### EXEMPLE 25 : 2-(4-Fluoro-benzènesulfonyl)-9-[(3-morpholin-4-yl-proprylcarbamoyl)-méthyl]-2,3,4,9-tétrahydro-1H-β-carboline-(3R)-(N-hydroxy)carboxamide

Le produit attendu est obtenu selon le même procédé décrit dans l'exemple 1, en utilisant au stade D le chlorure de 4-fluorobenzènesulfonique comme réactif.

### EXEMPLE 26 : 2-[(4-Méthoxyphényl)sulfonyl]-9-{[3-(2-méthylpipéridina)-propylcarbamoyl]-méthyl}-2,3,4,9-tétrahydro-2H-β-carboline-(3R)-(N-hydroxy)carboxamide

Le produit attendu est obtenu selon le même procédé décrit dans l'exemple 1, en utilisant au stade G, le 1-(3-Aminopropyl)-2-pipecoline comme réactif.

### EXEMPLE 27 : 2-[(4-Méthoxyphényl)sulfonyl]-9-{[3-(hydroxysulfonyl)propylcarbamoyl]-méthyl}-2,3,4,9-tétrahydro-1H-β-carboline-(3R)-(N-hydroxy)carboxamide

Le produit attendu est obtenu selon le même procédé décrit dans l'exemple 1, en utilisant au stade G, l'acide 3-Amino-1-propanesulfonique comme réactif.

### EXEMPLE 28 : 2-[(4-Méthoxyphénylsulfonyl]-9-{[2-(2-aminoéthoxy)éthyl-carbamoyl]-méthyl}-2,3,4,9-tétrahydro-1H-β-carboline-(3R)-(N-hydroxy) carboxamide

Le produit attendu est obtenu selon le même procédé décrit dans l'exemple 1, en utilisant au stade G, la 2-(2-aminoéthoxy)-1-éthanamine comme réactif.

### EXEMPLE 29 : 2-[(4-Méthoxyphényl)sulfonyl]-9-[(3-morpholine-4-yl-propylcarbamoyl)-éthyl]-2,3,4,9-tétrahydra-1H-β-carboline-(3R)-(N-hydroxy)carboxamide

Le produit attendu est obtenu selon le même procédé décrit dans l'exemple 1, en utilisant au stade A, le bromo-1-propanoate de tert-butyle comme réactif.

### ETUDE PHARMACOLOGIQUE DES COMPOSES DE L'INVENTION

### EXEMPLE 30 : Inhibition enzymatique des métalloprotéases

Les quatre enzymes humaines recombinantes MMP-1 (collagénase interstitielle), MMP-2 (gélatinase A de 72kda), MMP-3 (stromélysine 1) et MMP-9 (gélatinase B de 92kda) sont activées à l'AMPA (4-aminophénylmercuric acétate).
Les tests enzymatiques sont effectués avec un substrat peptido-mimétique :

DnpProChaGlyCys(Me)HisAlaLys(Nma)NH₂,

clivé entre la Glycine et la Cystéine pour produire un dérivé fluorescent (Anal. Biochem., 212, 58-64, 1993).
Les réactions conduites dans un tampon 50 mM Tris, 200 mM NaCl, 5 mM CaCl₂, 0,1 % Brij 35 à pH 7,7 sont initiées avec 20 µM de substrat dans un volume total de 100 µl à 37°C.
La fluorescence obtenue après six heures est lue en plaque 96-puits dans un fluorimètre équipé avec une combinaison de filtres de 340 nm et 440 nm pour l'excitation et l'émission. Lors de ces tests, les composés de l'invention ont présentés des IC₅₀ comprises entre 30 et 400 nM pour l'enzyme MMP-1, et entre 0,1 et 42 nM pour les enzymes MMP-2, MMP-3 et MMP-9. Plus spécifiquement, le composé de l'exemple 2 présente un IC₅₀ de 32,6 nM pour l'enzyme MMP-1, et le composé de l'exemple 11 présente respectivement des IC₅₀ de 1,6 nM, 3,1 nM et 0,1 nM pour les enzymes MMP-2, MMP-3 et MMP-9.

### EXEMPLE 31 : Angiogénèse in vitro

Des tronçons d'aorte thoracique de rats mâles Fischer 344 âgés de 8 à 12 semaines sont immergés dans un gel de collagène de type I selon la méthode de Nicosia et Ottinetti (1990). Après cinq jours de culture en milieu sans sérum, les préparations sont examinées au microscope et la formation de pseudo-vaisseaux quantifiée en terme de densité vasculaire après digitalisation et analyse d'image. A titre d'exemple, lors de test à 1 µM, les composés des exemples 2, 13 et 15 conduisent à une inhibition de la néovascularisation de 50 à 100 %.

### EXEMPLE 32 : Composition pharmaceutique

| Formule de préparation pour 1000 comprimés dosés à 10 mg : | |
|---|---|
| Composé de l'exemple 1 | 10 g |
| Hydroxypropylcellulose | 2 g |
| Amidon de blé | 10 g |
| Lactose | 100 g |
| Stéarate de magnésium | 3 g |
| Talc | 3 g |

## Revendications

1. Composés de formule (I) : 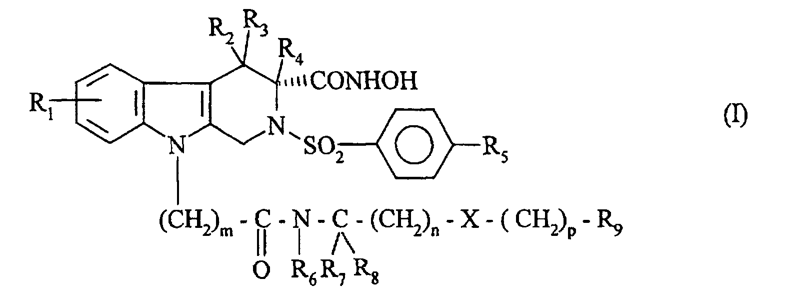 dans laquelle :
m représente un entier compris entre 1 et 4 inclus,
n et p, identiques ou différents, indépendamment l'un de l'autre, représentent un entier compris entre 0 et 4 inclus,
x représente un atome d'oxygène, de soufre ou une liaison simple,
R₁ représente un atome d'hydrogène, un atome d'halogène, un groupement alkyle (C₁-C₆) linéaire ou ramifié, trihalogénoalkyle (C₁-C₆) linéaire ou ramifié, hydroxy, alkoxy (C₁-C₆) linéaire ou ramifié ou un groupement trihalogénoalkoxy (C₁-C₆) linéaire ou ramifié,
R₂, R₃, R₄, identiques ou différents, indépendamment l'un de l'autre, représentent un atome d'hydrogène ou un groupement alkyle (C₁-C₆) linéaire ou ramifié,
R₅ représente un atome d'hydrogène, un atome d'halogène, un groupement alkoxy (C₁-C₆) linéaire ou ramifié, aryloxy ou un groupement hétéroaryloxy,
R₆, R₇, R₈, identiques ou différents, indépendamment l'un de l'autre, représentent un atome d'hydrogène, un groupement alkyle (C₁-C₆) linéaire ou ramifié, ou forment ensemble (R₆ et R₇ ou R₆ et R₈), avec l'atome d'azote portant le groupement R₆ et l'atome de carbone portant les groupements R₇ et R₈, un hétérocycle éventuellement substitué, le groupement restant (respectivement R₈ ou R₇) prenant alors une des définitions précédemment citées,
R9 représente l'un quelconque des groupements suivants :
* -SO₃H,
* -CO₂R₁₀ dans lequel R₁₀ représente un atome d'hydrogène ou un groupement alkyle (C₁-C₆) linéaire ou ramifié,
* -CO-NR₁₁R₁₂ dans lequel R₁₁ et R₁₂, identiques ou différents, représentent un atome d'hydrogène, un groupement alkyle (C₁-C₆) linéaire ou ramifié, ou R₁₁ et R₁₂ forment ensemble, avec l'atome d'azote qui les porte, un hétérocycle éventuellement substitué,
* -NR₁₃R₁₄ dans lequel R₁₃ et R₁₄, identiques ou différents, représentent un atome d'hydrogène, un groupement alkyle (C₁-C₆) linéaire ou ramifié, ou R₁₃ et R₁₄ forment ensemble , avec l'atome d'azote qui les porte, un hétérocycle éventuellement substitué,
étant entendu que :
- par groupement aryle, on comprend un groupement phényle, naphtyle, dihydronaphtyle, tétrahydronaphtyle, chacun de ces groupements étant éventuellement substitué par un ou plusieurs groupements identiques ou différents choisis parmi halogène, alkyle (C₁-C₆) linéaire ou ramifié, trihalogénoalkyle (C₁-C₆) linéaire ou ramifié, alkoxy (C₁-C₆) linéaire ou ramifié, trihalogénoalkoxy (C₁-C₆) linéaire ou ramifié ou hydroxy,
- par groupement hétéroaryle, on comprend un groupement aryle, éventuellement substitué par un ou plusieurs groupements, tels que définis précédemment, et contenant un, deux ou trois hétéroatomes, identiques ou différents, choisis parmi oxygène, azote ou soufre,
- par hétérocycle, on comprend un groupement mono ou bicyclique saturé ou insaturé, de 4 à 7 chaînons contenant un, deux ou trois hétéroatomes, identiques ou différents, choisis parmi oxygène, azote ou soufre, ledit hétérocycle pouvant être éventuellement substitué par un ou plusieurs groupements identiques ou différents choisis parmi halogène, alkyle (C₁-C₆) linéaire ou ramifié, alkoxy (C₁-C₆) linéaire ou ramifié, hydroxy, amino (éventuellement substitué par un ou deux groupements, identiques ou différents, alkyle (C₁-C₆) linéaire ou ramifié) ou par un hétérocycle,
leurs isomères optiques ainsi que leurs sels d'addition à un acide ou à une base pharmaceutiquement acceptable.

2. Procédé de préparation des composés de formule (I) selon la revendication 1 **caractérisé en ce que** l'on utilise comme produit de départ un dérivé du D-tryptophane, sous forme racémique ou d'énantiomère pur, de formule (II): 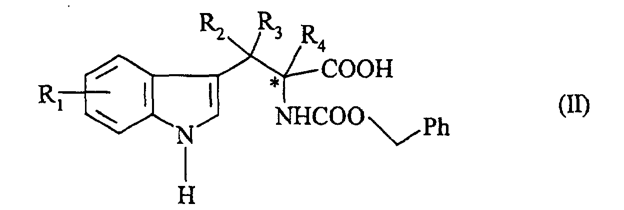 dans laquelle R₁, R₂, R₃ et R₄ sont tels que définis dans la formule (I),
dont on substitue la fonction amine du cycle indole par un dérivé halogéné de formule (III) :
Z - (CH₂)ₘ - CO₂Ra (III)
dans laquelle m a la même définition que dans la formule (I), Z représente un atome d'halogène et Ra représente un groupement alkyle (C₁-C₆) linéaire ou ramifié,
pour conduire aux composés de formule (IV) : 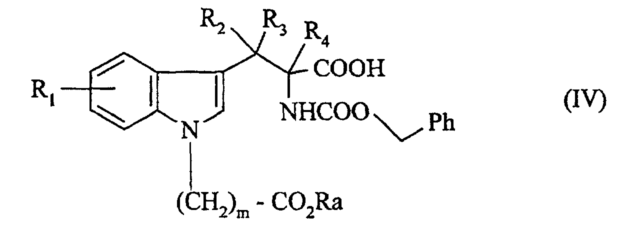 dans laquelle R₁, R₂, R₃, R₄, Ra et m sont tels que définis précédemment,
composés de formule (IV) dont on déprotège la fonction amine de la chaîne latérale par une réaction d'hydrogénation catalytique pour conduire aux composés de formule (V) : 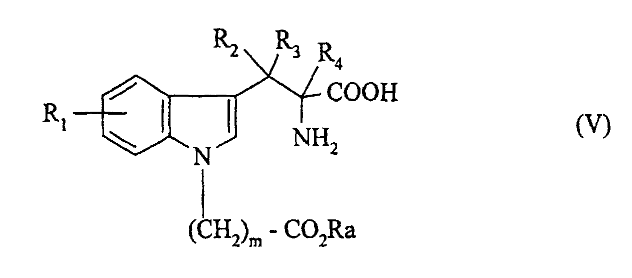 dans laquelle R₁, R₂, R₃, R₄, Ra et m sont tels que définis précédemment,
que l'on cyclise en présence d'un acide fort et de formaldéhyde, pour obtenir les composés de formule (VI): 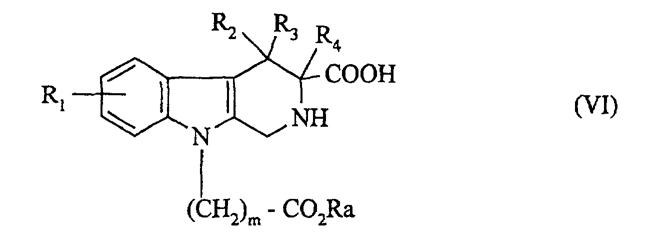 dans laquelle R₁, R₂, R₃, R₄, Ra et m sont tels que définis précédemment,
composés de formule (VI) que l'on condense en milieu basique avec un dérivé halogéné de formule (VII) : 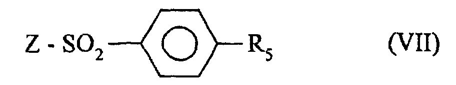 dans laquelle R₅ a la même signification que dans la formule (I) et Z représente un atome d'halogène,
pour conduire aux composés de formule (VIII) : 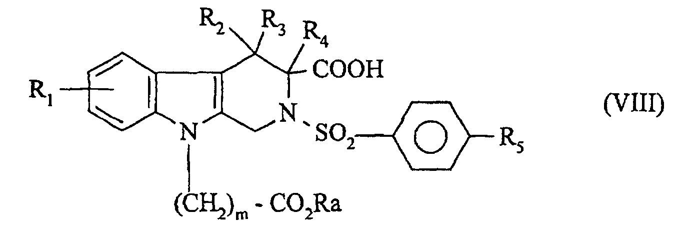 dans laquelle R₁, R₂, R₃, R₄, R₅, Ra et m sont tels que définis précédemment,
que l'on condense en milieu basique et en présence de dicyclohexylcarbodiimide avec une hydroxylamine-O-substituée de formule (IX) :
H₂N - O - R₁₅ (IX)
dans laquelle R₁₅ représente un groupement allyle ou benzyle,
pour obtenir les composés de formule (X): 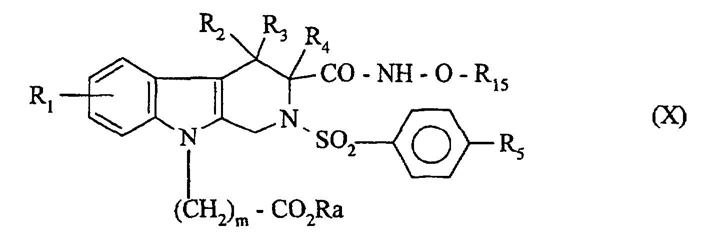 dans laquelle R₁, R₂, R₃, R₄, R₅, R₁₅, Ra et m sont tels que définis précédemment,
dont on déprotège la fonction acide carboxylique, en présence par exemple d'acide trifluoroacétique, pour conduire aux composés de formule (XI): 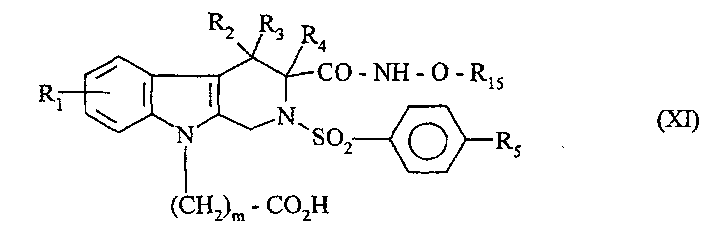 dans laquelle R₁, R₂, R₃, R₄, R₅, R₁₅ et m sont tels que définis précédemment,
que l'on condense avec une amine de formule (XII): 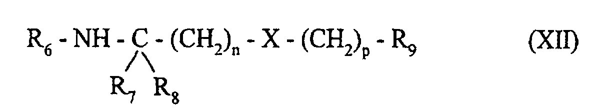 dans laquelle R₆, R₇, R₈, R₉, n et p ont la même définition que dans la formule (**I**),
pour conduire aux composés de formule (XIII): 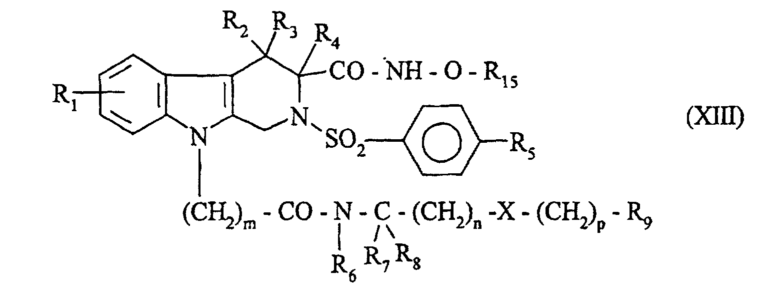 dans laquelle R₁, R₂, R₃, R₄, R₅, R₆, R₇, R₈, R₉ et R₁₅ sont tels que définis précédemment, ainsi que m, n et p,
composés de formule (XIII) dont la fonction hydroxylamine est déprotégée, pour conduire aux composés de formule (I): 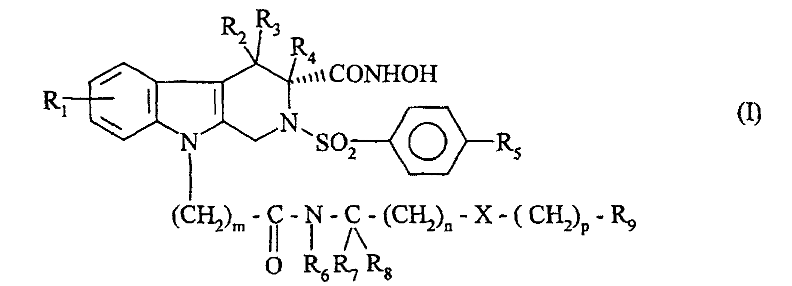 dans laquelle R₁, R₂, R₃, R₄, R₅, R₆, R₇, R₈, R₉, m, n et p sont tels que définis précédemment,
composés de formule (I) que l'on purifie, le cas échéant, selon une technique classique de purification, dont on sépare éventuellement les isomères selon une technique classique de séparation et que l'on transforme, si on le souhaite, en ses sels d'addition à un acide ou à une base pharmaceutiquement acceptable.

3. Compositions pharmaceutiques contenant comme principe actif au moins un composé selon la revendication 1, seul ou en combinaison avec un ou plusieurs excipients ou véhicules inertes, non toxiques, pharmaceutiquement acceptables.

4. Compositions pharmaceutiques selon la revendication 3 contenant au moins un principe actif selon la revendication 1 utiles comme inhibiteurs de métalloprotéases.

## Patentansprüche

1. Verbindungen der Formel (I): 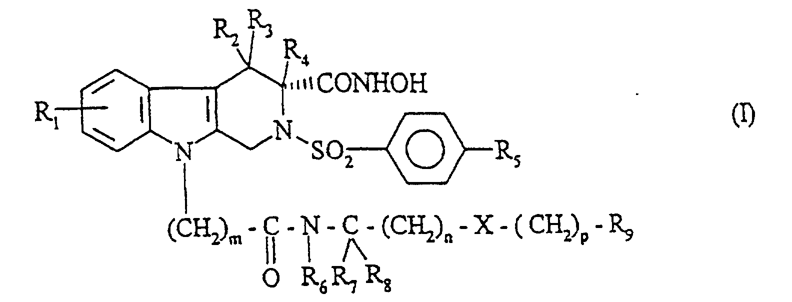 in der:
m eine ganze Zahl mit einem Wert zwischen 1 und 4 einschließlich darstellt,
n und p, , die gleichartig oder verschieden sind, unabhängig voneinander ganze Zahlen zwischen 0 und 4 einschließlich bedeuten,
x ein Sauerstoffatom, ein Schwefelatom oder eine Einfachbindung darstellt,
R₁ ein Wasserstoffatom, ein Halogenatom, eine geradkettige oder verzweigte (C₁-C₆)-Alkylgruppe, eine geradkettige oder verzweigte (C₁-C₆)-Trihalogenalkylgruppe, eine Hydroxygruppe, eine geradkettige oder verzweigte (C1-C₆)-Alkoxygruppe oder eine geradkettige oderverzweigte (C₁-C₆)-Trihalogenalkoxygruppe bedeutet,
R₂, R₃, R₄, die gleichartig oder verschieden sind, unabhängig voneinander Wasserstoffatome oder geradkettige oder verzweigte (C₁-C₆)-Alkylgruppen darstellen,
R₅ ein Wasserstoffatom, ein Halogenatom, eine geradkettige oder verzweigte (C₁-C₆)-Alkoxygruppe, eine Aryloxygruppe oder eine Heteroaryloxygruppe darstellt,
R₆, R₇, R₈, die gleichartig oder verschieden sind, unabhängig voneinander Wasserstoffatome, geradkettige oder verzweigte (C₁-C₆)-Alkylgruppen bedeuten oder gemeinsam (R₆ und R₇ oder R₆ und R₈) mit dem die Gruppe R₆ tragenden Stickstoffatom und dem die Gruppen R₇ und R₈ tragenden Kohlenstoffatom einen gegebenenfalls substituierten Heterocyclus bilden, wobei die verbleibende Gruppe (R₈ bzw. R₇) eine der angegebenen Bedeutungen besitzt,
R₉ irgendeine der folgenden Gruppen darstellt:
* -SO₃H,
* -CO₂R₁₀, worin R₁₀ ein Wasserstoffatom oder eine geradkettige oder verzweigte (C₁-C₆)-Alkylgruppe darstellt,
* -CO-NR₁₁R₁₂, worin R₁₁ und R₁₂, die gleichartig oder verschieden sind, Wasserstoffatome, geradkettige oder verzweigte (C₁-C₆)-Alkylgruppen bedeuten oder R₁₁ und R₁₂ gemeinsam mit dem sie tragenden Stickstoffatom einen gegebenenfalls substituierten Heterocyclus bilden,
* -NR₁₃R₁₄, worin R₁₃ und R₁₄, die gleichartig oder verschieden sind, Wasserstoffatome, geradkettige oder verzweigte (C₁-C₆)-Alkylgruppen bedeuten oder R₁₃ und R₁₄ gemeinsam mit dem sie tragenden Stickstoffatom einen gegebenenfalls substituierten Heterocyclus bilden,
wobei es sich versteht, daß:
- unter einer Arylgruppe eine Phenyl-, Naphthyl-, Dihydronaphthyl-, Tetrahydronaphthyl-gruppe zu verstehen ist, wobei jede dieser Gruppen gegebenenfalls durch eine oder mehrere gleichartige oder verschiedene Gruppen ausgewählt aus Halogen, geradkettigem oder verzweigtem (C₁-C₆)-Alkyl, geradkettigem oder verzweigtem (C₁-C₆)-Trihalogenalkyl, geradkettigem oder verzweigtem (C₁-C₆)-Alkoxy, geradkettigem oder verzweigtem (C₁-C₆)-Trihalogenalkoxy oder Hydroxy substituiert sind,
- unter einer Heteroarylgruppe eine Arylgruppe zu verstehen ist, die gegebenenfalls durch eine oder mehrere Gruppen, wie sie oben definiert worden sind, substituiert ist und die ein, zwei oder drei gleichartige oder verschiedene Heteroatome ausgewählt aus Sauerstoff, Stickstoff oder Schwefel enthält,
- unter einem Heterocyclus eine mono- oder bicyclische, gesättigte oder ungesättigte Gruppe mit 4 bis 7 Kettengliedern zu verstehen ist, die ein, zwei oder drei gleichartige oder verschiedene Heteroatome ausgewählt aus Sauerstoff, Stickstoff oder Schwefel enthält, wobei der Heterocyclus gegebenenfalls durch eine oder mehrere gleichartige oder verschiedene Gruppen ausgewählt aus Halogen, geradkettigem oder verzweigtem (C₁-C₆)-Alkyl, geradkettigem oder verzweigtem (C₁-C₆)-Alkoxy, Hydroxy, (gegebenenfalls durch eine oder zwei gleichartige oder verschiedene, geradkettige oder verzweigte (C₁-C₆)-Alkylgruppen substituiertem) Amino oder einen Heterocyclus substituiert sein kann,
- deren optische Isomere sowie deren Additionssalze mit einer pharmazeutisch annehmbaren Säure oder Base.

2. Verfahren zur Herstellung der Verbindungen der Formel (I) nach Anspruch 1, **dadurch gekennzeichnet, daß** man als Ausgangsprodukt ein D-Tryptophanderivat in Form des Racemats oder des reinen Enantiomeren der Formel (II) verwendet: 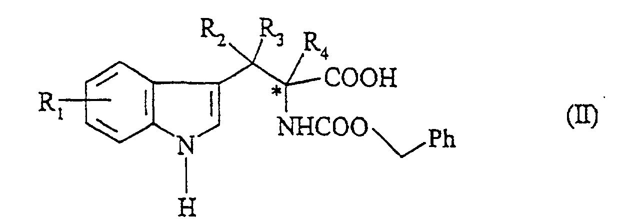 in der R₁, R₂, R₃ und R₄ die bezüglich der Formel (I) angegebenen Bedeutungen besitzen,
deren Aminfunktion des Indolrings man mit einem Halogenderivat der Formel (III):
Z - (CH₂)ₘ - CO₂Ra (III)
in der m die bezüglich der Formel (I) angegebenen Bedeutungen besitzt, Z ein Halogenatom und Ra eine geradkettige oder verzweigte (C₁-C₆)-Alkylgruppe bedeuten, substituiert
zur Bildung der Verbindungen der Formel (IV): 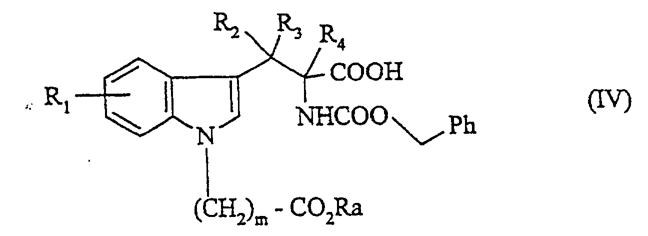 in der R₁, R₂, R₃, R₄, Ra und m die oben angegebenen Bedeutungen besitzen, von welchen Verbindungen der Formel (IV) man die Schutzgruppe der Aminfunktion der Seitenkette durch eine katalytische Hydrierungsreaktion abspaltet zur Bildung der Verbindungen der Formel (V): 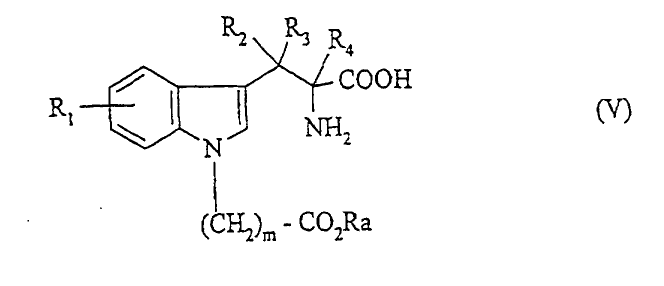 in der R₁, R₂, R₃, R₄, Ra und m die oben angegebenen Bedeutungen besitzen, welche man in Gegenwart einer starken Säure und von Formaldehyd cyclisiert zur Bildung der Verbindungen der Formel (VI): 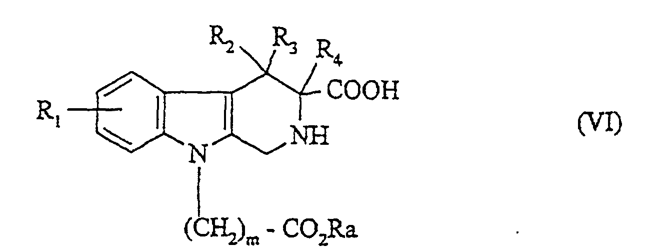 in der R₁, R₂, R₃, R₄, Ra und m die oben angegebenen Bedeutungen besitzen, welche Verbindungen der Formel (VI) man in basischem Medium mit einem Halogenderivat der Formel (VII): 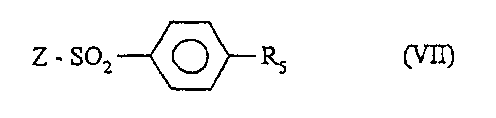 in der R₅ die bezüglich der Formel (I) angegebenen Bedeutungen besitzt und Z ein Halogenatom darstellt, kondensiert,
zur Bildung der Verbindungen der Formel (VIII): 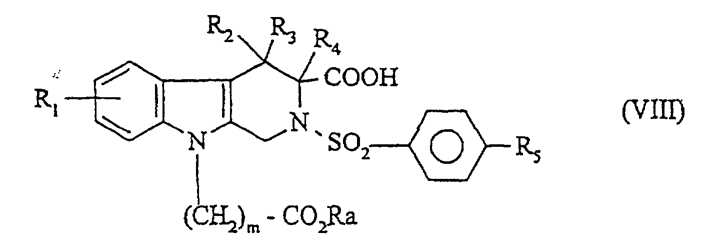 in der R₁, R₂, R₃, R₄, R₅, Ra und m die oben angegebenen Bedeutungen besitzen,
welche man in basischem Medium und in Gegenwart von Dicyclohexylcarbodiimid mit einem O-substituierten Hydroxylamin der Formel (IX):
H₂N - O - R₁₅ (IX)
in der R₁₅ eine Allyl- oder Benzylgruppe bedeutet, kondensiert, zur Bildung der Verbindungen der Formel (X): 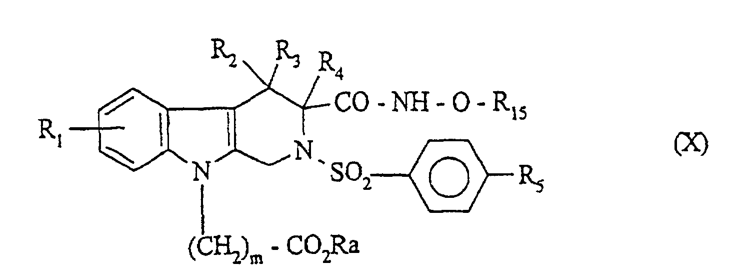 in der R₁, R₂, R₃, R₄, R₅, R₁₅, Ra und m die oben angegebenen Bedeutungen besitzen,
von welchen man die Schutzgruppe der Carbonsäurefunktion in Gegenwart von beispielsweise Trifluoressigsäure abspaltet zur Bildung der Verbindungen der Formel (XI): 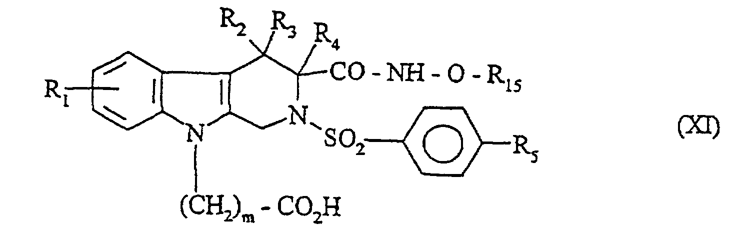 in der R₁, R₂, R₃, R₄, R₅, R₁₅ und m die oben angegebenen Bedeutungen besitzen,
welche man mit einem Amin der Formel (XII): 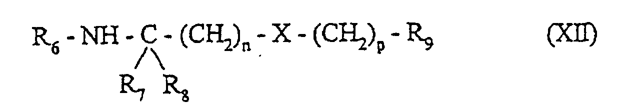 in der R₆, R₇, R₈, R₉, n und p die bezüglich der Forme (I) angegebenen Bedeutungen besitzen, kondensiert,
zur Bildung der Verbindunge der Formel (XIII): 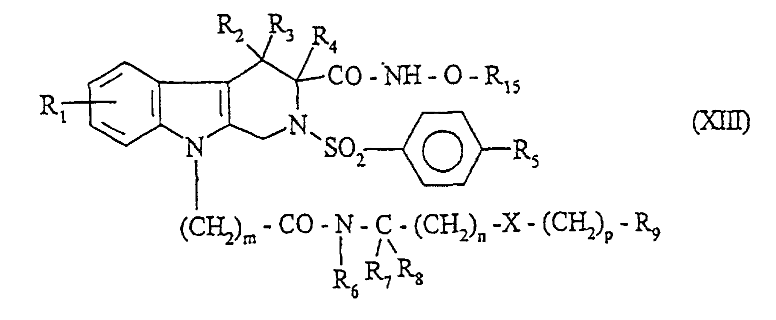 in der R₁, R₂, R₃, R₄, R₅, R₆, R₇, R₈, R₉ und R₁₅ die oben angegebenen Bedeutungen besitzen, ebenso wie m, n und p,
von welchen Verbindungen der Formel (XIII) man die Schutzgruppe der Hydroxylaminfunktion abspaltet zur Bildung der Verbindungen der Formel (I): 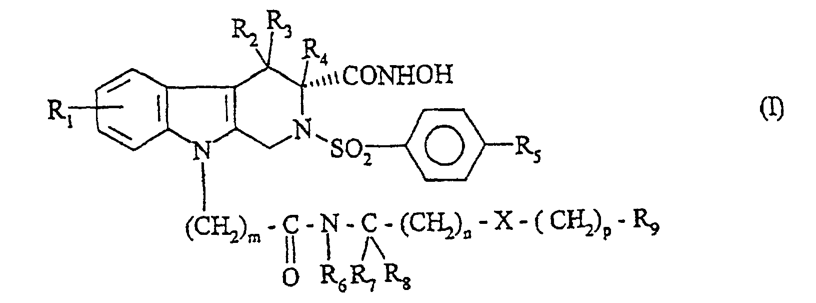 in der R₁, R₂, R₃, R₄, R₅, R₆, R₇, R₈, R₉, m, n und die oben angegebenen Bedeutungen besitzen,
welche Verbindungen der Formel (I) man gegebenenfalls mit Hilfe einer klassischen Reinigungsmethode reinigt, gegebenenfalls mit Hilfe einer klassischen Trennungsmethode in ihre Isomeren trennt und gewünschtenfalls mit einer pharmazeutisch annehbmaren Säure oder Base in ihre Additionssalze umwandelt.

3. Pharmazeutische Zubereitungen enthaltend als Wirkstoff mindestens eine Verbindung nach Anspruch 1 allein oder in Kombination mit einem oder mehreren inerten, nichttoxischen, pharmazeutisch annehmbaren Trägermaterialien oder Bindemitteln.

4. Pharmazeutische Zubereitungen nach Anspruch 3 enthaltend mindestens einen Wirkstoff nach Anspruch 1 als Inhibitoren von Metalloproteasen.

## Claims

1. Compounds of formula (I) : 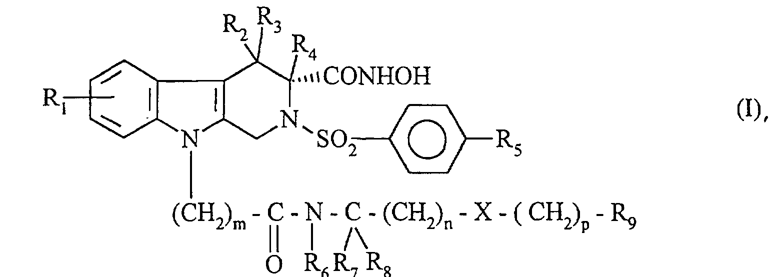 in which:
m represents an integer from 1 to 4 inclusive,
n and p, which may be the same or different, each represents independently of the other an integer from 0 to 4 inclusive,
X represents an oxygen or sulphur atom or a single bond,
R₁ represents a hydrogen atom, a halogen atom, a linear or branched (C₁-C₆)alkyl group, a linear or branched (C₁-C₆)trihaloalkyl group, a hydroxy group, a linear or branched (C₁-C₆)alkoxy group or a linear or branched (C₁-C₆)trihaloalkoxy group,
R₂, R₃ and R₄, which may be the same or different, each represents independently of the others a hydrogen atom or a linear or branched (C₁-C₆)alkyl group,
R₅ represents a hydrogen atom, a halogen atom, a linear or branched (C₁-C₆)alkoxy group, an aryloxy group or a heteroaryloxy group,
R₆, R₇ and R₈, which may be the same or different, each represents independently of the others a hydrogen atom or a linear or branched (C₁-C₆)alkyl group, or together (R₆ and R₇ or R₆ and R₈) form, with the nitrogen atom carrying the group R₆ and the carbon atom carrying the groups R₇ and R₈, an optionally substituted heterocycle, the remaining group (R₈ or R₇, respectively) then having one of the meanings given above,
R₉ represents any one of the following groups :
* -SO₃H,
* -CO₂R₁₀ in which R₁₀ represents a hydrogen atom or a linear or branched (C₁-C₆)alkyl group,
* -CO-NR₁₁R₁₂ in which R₁₁ and R₁₂, which may be the same or different, each represents a hydrogen atom or a linear or branched (C₁-C₆)alkyl group, or R₁₁ and R₁₂ together form, with the nitrogen atom carrying them, an optionally substituted heterocycle,
* -NR₁₃R₁₄ in which R₁₃ and R₁₄, which may be the same or different, each represents a hydrogen atom or a linear or branched (C₁-C₆)alkyl group, or R₁₃ and R₁₄ together form, with the nitrogen atom carrying them, an optionally substituted heterocycle,
it being understood that:
- an aryl group is taken to mean a phenyl, naphthyl, dihydronaphthyl or tetrahydronaphthyl group, each of those groups being optionally substituted by one or more identical or different groups selected from halogen, linear or branched (C₁-C₆)alkyl, linear or branched (C₁-C₆)trihaloalkyl, linear or branched (C₁-C₆)alkoxy, linear or branched (C₁-C₆)trihaloalkoxy and hydroxy,
- a heteroaryl group is taken to mean an aryl group that is optionally substituted by one or more groups as defined above and that contains one, two or three identical or different hetero atoms selected from oxygen, nitrogen and sulphur,
- a heterocycle is taken to mean a saturated or unsaturated, mono- or bi-cyclic, 4- to 7-membered group containing one, two or three identical or different hetero atoms selected from oxygen, nitrogen and sulphur, it being possible for the said heterocycle optionally to be substituted by one or more identical or different groups selected from halogen, linear or branched (C₁-C₆)alkyl, linear or branched (C₁-C₆)alkoxy, hydroxy and amino (optionally substituted by one or two linear or branched (C₁-C₆)alkyl groups, which may be the same or different) or by a heterocycle,
their optical isomers, and addition salts thereof with a pharmaceutically acceptable acid or base.

2. Process for the preparation of compounds of formula (I) according to claim 1, **characterised in that** there is used as starting material a D-tryptophan compound, in racemic form or in the form of a pure enantiomer, of formula (II): 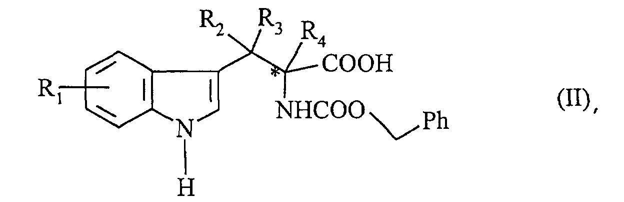 in which R₁, R₂, R₃ and R₄ are as defined for formula (I),
the amine function of the indole ring system of which is substituted by a halogenated compound of formula (III) :
Z - (CH₂)ₘ - CO₂Ra (III),
in which m is as defined for formula (I), Z represents a halogen atom and Ra represents a linear or branched (C₁-C₆)alkyl group,
to yield a compound of formula (IV): 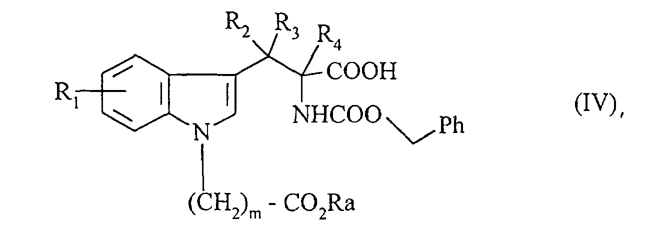 in which R₁, R₂, R₃, R₄, Ra and m are as defined above,
the amine function of the side chain of which compound of formula (IV) is deprotected by a catalytic hydrogenation reaction to yield a compound of formula (V): 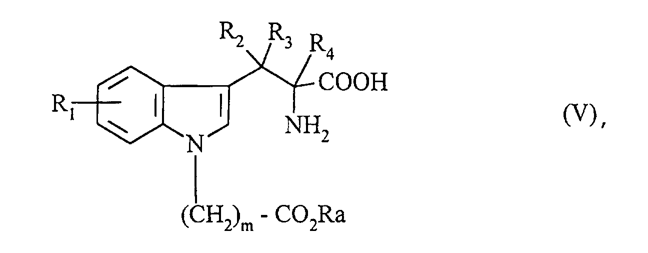 in which R₁, R₂, R₃, R₄, Ra and m are as defined above,
which is cyclised, in the presence of a strong acid and formaldehyde, to obtain a compound of formula (VI): 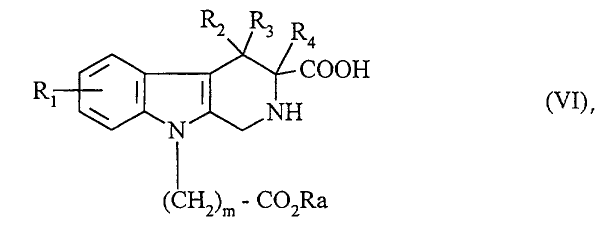 in which R₁, R₂, R₃, R₄, Ra and m are as defined above,
which compound of formula (VI) is condensed, in a basic medium, with a halogenate compound of formula (VII) : 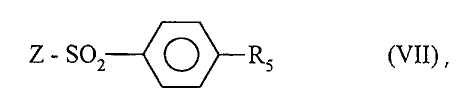 in which R₅ is as defined for formula (I) and Z represents a halogen atom, to yield a compound of formula (VIII): 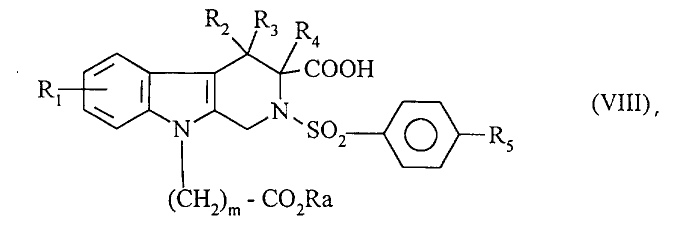 in which R₁, R₂, R₃, R₄, R₅, Ra and m are as defined above,
which is condensed, in a basic medium and in the presence of dicyclohexylcarbodiimide, with an O-substituted hydroxylamine of formula (IX):
H₂N - O - R₁₅ (IX),
in which R₁₅ represents an allyl or benzyl group,
to obtain a compound of formula (X): 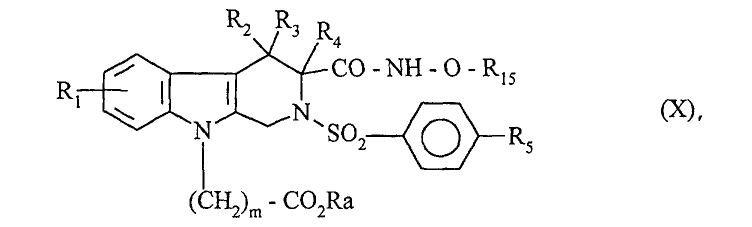 in which R₁, R₂, R₃, R₄, R₅, R₁₅, Ra and m are as defined above,
the carboxylic acid function of which is deprotected, in the presence, for example, of trifluoroacetic acid, to yield a compound of formula (XI): 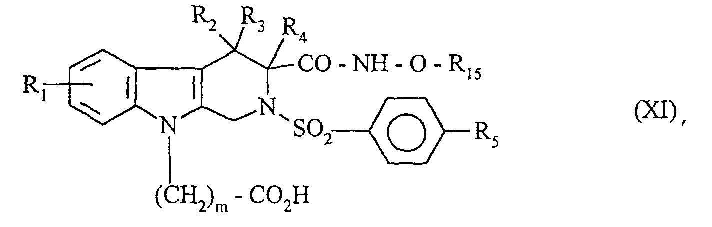 in which R₁, R₂, R₃, R₄, R₅, R₁₅ and m are as defined above, which is condensed with an amine of formula (XII): 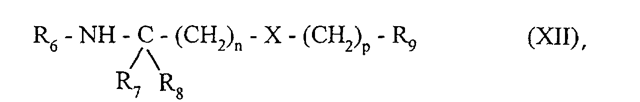 in which R₆, R₇, R₈, R₉, n and p are as defined for formula (I), to yield a compound of formula (XIII): 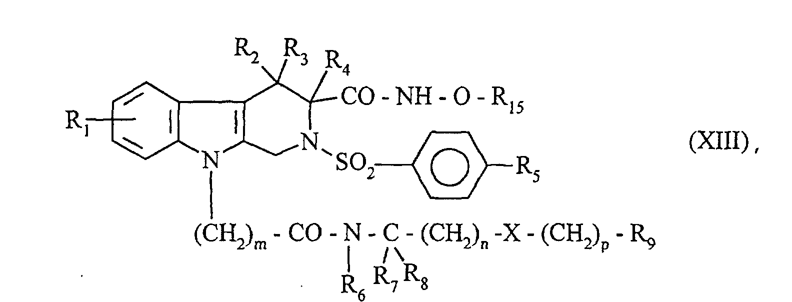 in which R₁, R₂, R₃, R₄, R₅, R₆, R₇, R₈, R₉ and R₁₅ are as defined above, as are m, n and p, the hydroxylamine function of which compound of formula (XIII) is deprotected to yield the compound of formula (I): 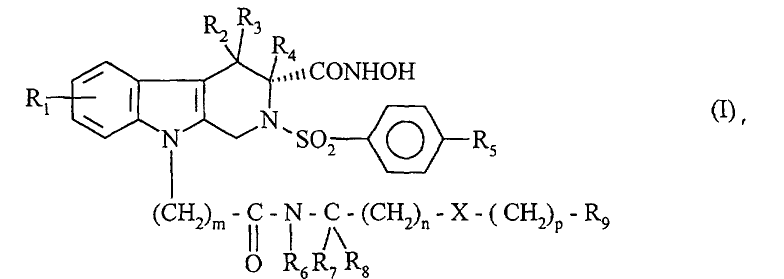 in which R₁, R₂, R₃, R₄, R₅, R₆, R₇, R₈, R₉, m, n and p are as defined above,
which compounds of formula (I) are, if necessary, purified according to a conventional purification technique, are, where appropriate, separated into their isomers according to a conventional separation technique and are, if desired, converted into their addition salts with a pharmaceutically acceptable acid or base.

3. Pharmaceutical compositions comprising as active ingredient at least one compound according to claim 1, on its own or in combination with one or more pharmaceutically acceptable, inert, non-toxic excipients or carriers.

4. Pharmaceutical compositions according to claim 3 comprising at least one active ingredient according to claim 1 for use as metalloprotease inhibitors.
